# EUROPÄISCHE PATENTSCHRIFT

(11) **EP 4 429 556 B1**
(45) Veröffentlichungstag und Bekanntmachung des Hinweises auf die Patenterteilung: **22.10.2025**
(21) Anmeldenummer: 22814359.0
(22) Anmeldetag: 08.11.2022
(51) Int. Cl.: A61B 6/03, A61B 3/10

(54) **WEITFELD-SWEPT-SOURCE-OCT UND -VERFAHREN FÜR BEWEGTE OBJEKTE**
WIDE-FIELD SWEPT-SOURCE OCT AND METHOD FOR MOVING OBJECTS
TCO À SOURCE BALAYÉE À CHAMP LARGE ET PROCÉDÉ DE DÉPLACEMENT D'OBJETS

(30) Priorität: 12.11.2021 DE 102021129555
(43) Veröffentlichungstag der Anmeldung: 18.09.2024
(73) Patentinhaber: Carl Zeiss Meditec AG, 07745 Jena (DE)
(72) Erfinder: BUBLITZ, Daniel, 07745 Jena (DE); SEIDEL, Dirk, 07745 Jena (DE)
(74) Vertreter: Patentanwälte Geyer, Fehners & Partner mbB
(86) Internationale Anmeldenummer: PCT/EP2022/081158
(87) Internationale Veröffentlichungsnummer: WO 2023/083822

(56) Entgegenhaltungen:
- US-A1- 2010 208 962
- US-A1- 2012 106 814
- US-B2- 8 731 268

## Beschreibung

Die Erfindung bezieht sich auf ein Weitfeld-Swept-Source-OCT und -Verfahren für bewegte Objekte, insbesondere die Vorderkammer des menschlichen Auges.

Ophthalmologische OCT-Systeme werden seit vielen Jahren zur Vermessung der menschlichen Retina und auch von Strukturen in der Vorderkammer des Auges eingesetzt. Ein bekanntes Vorderkammer-OCT-System wurde von Carl Zeiss in Form eines konfokalen Scanners entwickelt.

Für Vorderkammermessungen werden häufig OCT-Systeme, die für die Retinabildgebung entwickelt wurden, durch eine Vorsatzoptik angepasst. Da die Abbildungseigenschaften für die Vorderkammermessung sich von der zur Retinaerfassung unterscheiden und die OCT-Systeme für die Retina optimiert sind, ist die Abbildungsleistung in der Vorderkammer nicht optimal. Die wesentlichen Unterschiede bei der Abbildung dieser beiden Gewebe sind folgende:
1. Alle Bündel, die zur Retina gelangen, werden durch die Iris beschnitten, so dass für die Retina gedachte OCT-Systeme alle mit einer ähnlichen Pupillengröße von 1,0 bis 1,3 mm und daraus folgenden numerischen Aperturen von 0,04 im Maximum arbeiten. Dadurch ist die laterale Auflösung begrenzt, die Schärfentiefe, die den zugänglichen Tiefenbereich bei der Messung bestimmt, bleibt aber ausreichend für eine bildliche Darstellung der Retina bis in die durch die Streuung begrenzte Messtiefe von knapp einem Millimeter.
2. Die Gewebe in der Retina, speziell das retinale Pigmentepithel und die dahinter liegende Aderhaut, sind stark streuend. Um die dadurch verursachte Mehrfachstreuung zu minimieren, sind die meisten OCT-Systeme als voll konfokale Systeme ausgelegt. Da die OCT-Beleuchtungs- und Detektionswellen von der menschlichen Augenlinse abgebildet werden, die gewisse Aberrationen aufweist, kann auch in sehr kleinen Bildfeldern und Tiefenbereichen nur eine geringfügig bessere laterale Auflösung erreicht werden.
3. Die Gewebe in der Vorderkammer (Cornea, Kammerwasser, Iris und Augenlinse) sind in der Regel deutlich weniger streuend, sodass auch mit nichtkonfokalen Verfahren gearbeitet werden könnte. Da außerdem die Eindringtiefe der Lichtwellenfelder in das Auge nur gering sind, stellen dadurch verursachte Aberrationen auch signifikant weniger Probleme dar, so dass mit einigen Vorderkammersystemen mit numerischen Aperturen von über 0,2 und Auflösungen bis in den µm-Bereich gearbeitet werden kann. Die daraus resultierende Schärfentiefe liegt für die verschiedenen Applikationen zwischen einigen Mikrometern und einigen 10 Mikrometern. Der in der Vorderkammer zu visualisierende Tiefenbereich ist deutlich größer und beträgt zirka 6 mm im Gewebe (abhängig von der genauen Applikation).

Daher gibt es grundlegend zwei Optimierungen für konfokale Vorderkammeraufnahmen, einen konfokalen Scanner mit hoher Auflösung, der die Vorderkammer in sehr vielen Tiefenebenen von je etwa einer Schärfentiefe sequenziell vermisst und die Bilder kombiniert. Alternativ kann die numerische Apertur nA gesenkt werden, bis eine Schärfentiefe von zirka 8 mm in Luft erreicht wird, was einer nA = 0,007 bei 840 nm und einer daraus resultierenden lateralen Auflösung von etwa 73 µm bei 840 nm entspricht.

Es sind im Stand der Technik auch nichtkonfokale Weitfeld-OCT-Systeme bekannt, die mit einer voll kohärenten Beleuchtung realisiert werden. Da diese Systeme voll kohärent und gleichzeitig nicht konfokal sind, können die detektierten Wellen rechnerisch in jede Probenebene nachträglich propagiert/fokussiert werden und damit der zugängliche Tiefenbereich von der lateralen Auflösung teilweise entkoppelt werden. Derartige Verfahren und Kohärenztomographen sind aus der DE 10 2014 115 157 A1 bekannt. Ähnliche Ansätze finden sich auch in der DE 10 2014 115 153 A1, DE 10 2014 115 155 A1, DE 10 2015 101 251 A1 und WO 2017/137567 A1.

In DE 10 2018 13 0396 A1 ist ein Verfahren der optischen Kohärenz Tomographie beschrieben, das mit einer gestreuten, voll kohärenten Beleuchtungswelle beleuchtet wird. Es sind auch spezielle Ausgestaltungen für eine Weitfeld-Bildaufnahme mit einem 2D-ortsaufgelösten Sensor dargestellt. Das beschriebene Verfahren wird bevorzugt für die Volumenbildgebung an biologischem Gewebe, wie z. B. dem menschlichen Auge angewendet. Es könnte darüber hinaus aber auch an technischen Proben eingesetzt werden.

Die Beleuchtung eines holoskopischen Systems mit einer gestreuten, aber voll kohärenten Beleuchtungswelle bietet zwei entscheidende Vorteile. Durch die gestreute Welle können deutlich höhere Beleuchtungsintensitäten eingestrahlt werden, ohne Schäden am Auge zu riskieren. Dadurch kann die Aufnahmegeschwindigkeit erhöht werden, da mehr Licht zur Verfügung steht. Dieser Vorteil tritt in Vorderkammersystemen nicht so stark auf, da hier auch durch eine angepasste Beleuchtungswelle, der Anteil der Strahlung, der durch die Iris tritt und bis zur Retina gelangt, beeinflusst werden kann. Außerdem können derartige Systeme auch prinzipiell mit Wellenlängen im Bereich von 1,3 µm bis 1,55 µm beleuchtet werden, die aufgrund der hohen Wasserabsorption des Glaskörpers nicht bis zur Retina transmittieren könnten.

Als zweiter Vorteil der Streuwellenbeleuchtung ist zu nennen, dass mehrfach gestreute Störlichtanteile von einfach gestreuten Signallichtanteilen unterschieden werden können und damit der Kontrast der Aufnahmen in stark streuenden Geweben verbessert werden kann. Auch dieser Vorteil kommt in den wenig streuenden Geweben der Vorderkammer kaum zur Geltung.

Je nach Größe des zu vermessenden Bildfeldes und der zu erreichenden lateralen Auflösungen können sehr große Pixelauflösungen resultieren, die mit verfügbaren schnellen Kamerasensoren nicht mehr erreicht werden können. In diesen Fällen wird das Bildfeld in mehreren Teilbildern aufgenommen, die zu einem Gesamtbild verrechnet/kombiniert werden. Durch Abschattungseffekte an den Grenzen der Teilbildfelder müssen die Teilbilder mit einem gewissen Überlapp aufgenommen werden, der sich am Verhältnis aus zu messendem Tiefenbereich und Schärfentiefe bemisst. Um unter diesen Umständen eine insgesamt sehr effiziente Messung zu erreichen, ist es besonders bevorzugt, das Gesamtbild aus so wenigen und so groß wie möglichen Teilfeldern zusammenzustellen.

Die Aufnahme der verschiedenen Wellenlängen mit einer 2D-Kamera zur Berechnung der Tiefenfunktion setzt eine kohärente, d. h. phasenstarre Aufnahme voraus. Restbewegungen des Auges, wenn wie applikativ bevorzugt ohne Kontaktglas gearbeitet wird, begrenzen die Zeit, in der diese Phasenkorrelation aufrechterhalten werden kann auf zirka 10 bis 25 ms. Es gibt im Stand der Technik Verfahren (z. B. EP 3 517 021 A1), die aus den Teilaufnahmen auch über Zeitspannen von unter einer Sekunde bei Retinaaufnahmen auftretende Bewegungen schätzen und numerisch bei der Auswertung korrigieren können. Vorderkammerbilder sind aber gegenüber Retinaaufnahmen weniger stark räumlich strukturiert, so dass diese Ansätze nicht sicher übertragen werden können.

Möchte man nur die Cornea hochauflösend vermessen, so benötigt man eine Wellenlängendurchstimmung über mindestens etwa 50 unterschiedliche Wellenlängenbilder, wenn man durch eine geeignete Beleuchtung mit einer gewölbten Zerodelayfläche arbeitet. Möchte man die gesamte Vorderkammer darstellen, benötigt man die Wellenlängendurchstimmung über etwa 400 Wellenlängenbilder, die alle innerhalb von etwa 25 ms aufgenommen werden müssten.

Für diese Bildratenanforderungen sind sehr teure Hochgeschwindigkeitskameras wie beispielsweise die Photron-SAZ Kamera geeignet, die Frameraten im Bereich bis 20 000 fps bei etwa 1 MPixel Auflösung erreichen. Diese Kamerasysteme sind aber nicht mit ausreichend guten Pixelauflösungen verfügbar und deshalb insgesamt wieder nicht geeignet.

Der Erfindung liegt deshalb die Aufgabe zu Grunde, ein beschleunigtes voll kohärentes Weitfeld-OCT-System bereitzustellen, wobei der Kamerasensor mit hoher Auflösung (also Pixelzahl) arbeitet, aber keine Geschwindigkeiten über 1000 fps erreichen muss. Insbesondere soll die Geschwindigkeit des Systems für Messungen der Vorderkammer des menschlichen Auges ohne Kontaktglas geeignet sein.

Die Erfindung ist in den unabhängigen Ansprüchen definiert. Die abhängigen Ansprüche betreffen bevorzugte Weiterbildungen.

Der Weitfeld-Swept-Source-OCT und das Verfahren dienen zur Abbildung eines bewegten Objekts, insbesondere der Vorderkammer des menschlichen Auges. Das Verfahren weist folgende Schritte auf:
- Bereitstellen von in der Wellenlänge durchgestimmter Beleuchtungsstrahlung, die einzelne Beleuchtungspulse unterschiedlicher Schwerpunktwellenlänge aufweist. Die Beleuchtungspulse werden als Serie von Beleuchtungspulspaaren abgegeben, die jeweils aus einem ersten Beleuchtungspuls und einem zweiten Beleuchtungspuls bestehen. In den Beleuchtungspulspaaren unterscheidet sich die Schwerpunktwellenlänge des ersten Beleuchtungspulses von der Schwerpunktwellenlänge des zweiten Beleuchtungspulses. In mehreren der Beleuchtungspulspaare wird eine Schwerpunktwellenlänge eines der Beleuchtungspulse eines vorhergehenden der Beleuchtungspulspaare wiederholt.
- Beleuchten des Objekts mit der Beleuchtungsstrahlung, wobei die Beleuchtungsstrahlung im Objekt als Messstrahlung rückgestreut oder -reflektiert wird und Abbilden der vom beleuchteten Objekt kommenden Messstrahlung auf einen 2D-Detektor. Das Beleuchten erfolgt bevorzugt durch kohärente Weitfeldbeleuchtung.
- Betreiben des Detektors gemäß einem Bildaufnahmezyklus umfassend eine Abfolge von Belichtungsintervallen und Ausleseintervallen.
- Synchronisieren der Beleuchtungspulse und der Abfolge von Belichtungsintervallen und Ausleseintervallen derart, dass die Beleuchtungspulspaare um jedes zweite der Ausleseintervalle der Abfolge herum gruppiert sind und für jedes Beleuchtungspulspaar der erste Beleuchtungspuls während eines letzten Drittels eines der Belichtungsintervalle und der zweite Beleuchtungspuls während eines ersten Drittels des folgenden der Belichtungsintervalle abgegeben wird.
- Auslesen von Bilddaten vom Detektor für jedes Belichtungsintervall und Zuordnen der Bilddaten zu den Schwerpunktwellenlängen des im jeweiligen Belichtungsintervall abgegebenen Beleuchtungspulses. Es werden Bildpaare entsprechend den Beleuchtungspulspaaren erzeugt.
- Ermitteln von Änderungen zwischen den Bilddaten der über die Beleuchtungspulspaare hinweg wiederholten Beleuchtungspulse mit gleicher Schwerpunktwellenlänge und Auswerten der Bilddaten und Verwenden der Änderungen zum Korrigieren von Bewegungen des Objektes in den Bilddaten.

Der Weitfeld-Swept-Source-OCT ist zur Umsetzung des Verfahrens entsprechend ausgebildet. Er umfasst:
- eine Strahlungsquelle, die konfiguriert ist, die in der Wellenlänge durchgestimmte Beleuchtungsstrahlung abzugeben, welche die einzelnen Beleuchtungspulse unterschiedlicher Schwerpunktwellenlänge aufweist, wobei die Beleuchtungspulse gemäß dem Verfahren als Serie von Beleuchtungspulspaaren mit den oben erläuterten Schwerpunktwellenlängen abgegeben werden.
- den hier auch als Kamera bezeichneten 2D-Detektor, der den Bildaufnahmezyklus umfassend die Abfolge von Belichtungsintervallen und Ausleseintervallen ausführt.
- einen Strahlengang zum Beleuchten des Objekts mit der Beleuchtungsstrahlung und zum Abbilden des beleuchteten Objekts auf den 2D-Detektor. Das Beleuchten erfolgt bevorzugt als kohärente Weitfeldbeleuchtung.
- eine Steuereinrichtung, die das Verfahren umsetzt. Sie steuert den 2D-Detektor und die Strahlungsquelle an und synchronisiert diese, wie zum Verfahren erwähnt. Sie erzeugt damit die genannten Bildpaare, Ermitteln von Änderungen zwischen den Bilddaten der über die Beleuchtungspulspaare hinweg wiederholten Beleuchtungspulse mit gleicher Schwerpunktwellenlänge und nutzt diese zum Referenzieren beim Auswerten der Bilddaten und korrigiert dadurch Bewegungen des Objektes in den Bilddaten.

Das Verfahren wird hier anhand einer Vorderkammeraufnahme erklärt werden, die Beschreibung gilt aber für alle medizinischen und technischen Weitfeld-SS-OCT-Systeme, bei denen Objektbewegungen eine kohärente Aufnahme limitieren. Insbesondere ist die vorgestellte Lösung auch für die Volumenbildgebung streuender Medien für mikroskopische in-vitro Anwendungen geeignet. Weiter gelten für das Verfahren beschriebene Aspekte gleichermaßen für den OCT und umgekehrt.

Direkt aufeinanderfolgende Beleuchtungspulspaare sind um ein Vielfaches des Abstandes, den die beiden Beleuchtungspulse im Beleuchtungspulspaar voneinander haben, beabstandet. Bevorzugt beträgt der Abstand aufeinanderfolgender Beleuchtungspulspaare mindestens die Dauer einer Sequenz von Belichtungsintervall und Ausleseintervall, bevorzugt mindestens das 1 1/3-fache, besonders bevorzugt mindestens das 1,5-fache der Dauer. Der Abstand jeweils durch das Ende des vorherigen Beleuchtungspulses und den Beginn des nächsten Beleuchtungspuls definiert.

Jedem Beleuchtungspulspaar ist ein Bildpaar, nachfolgend auch als Doppelbild bezeichnet, aus zwei Einzelbildern zugeordnet, denen jeweils die Schwerpunktwellenlänge des entsprechenden Beleuchtungspulses des Beleuchtungspulspaares zugeordnet ist. In mehreren Beleuchtungspulspaaren wird eine Schwerpunktwellenlänge eines der Beleuchtungspulse eines vorhergehenden der Beleuchtungspulspaare wiederholt. Diese definierte Struktur erlaubt eine Bewegungskorrektur über die Bildpaare hinweg, da für genau ein Einzelbild jedes Bildpaares in mind. einem anderen Bildpaar ein Einzelbild vorhanden ist, dem dieselbe Schwerpunktwellenlänge zugeordnet ist. Damit wird für diese Bildpaare aus einem Vergleich der Einzelbilder, denen dieselbe Schwerpunktwellenlänge zugeordnet ist, eine Objektbewegung ermittelt und für die Auswertung der anderen beiden Einzelbilder der Bildpaare korrigiert.

Diese Korrektur ist besonders einfach, wenn in mehreren Beleuchtungspulspaaren genau eine Schwerpunktwellenlänge eines der Beleuchtungspulse des unmittelbar vorhergehenden der Beleuchtungspulspaare wiederholt wird.

Dies realisiert z. B. Beleuchtungspulspaare (und damit Bildpaare) mit einer Wellenlängenfolge (lambda-0, lambda-1), (lambda-1, lambda-2), (lambda-2, lambda-3) usw. Allgemein genügen die Elemente der Folge aufs Beleuchtungspulspaaren dann der rekursiven Definition (lambda-k, lambda-k+1) mit Laufindex k von 1 bis n. Natürlich kann auch immer dieselbe Schwerpunktwellenlänge als Referenzschwerpunktwellenlänge wiederholt werden. Das liefert die rekursiven Definition (lambda-0, lambda-k) mit Laufindex k von 1 bis n und lambda-0 als Referenzschwerpunktwellenlänge. Beide Varianten (lambda-0, lambda-k) und (lambda-k, lambda-k+1) sind mathematisch äquivalent. Technisch ist die erstgenannte Variante zu bevorzugen, weil sie nur einen durchstimmbaren Laser benötigt. Da das lambda-0 dann auch einen größeren Wellenlängenabstand zu allen anderen Wellenlängen lambda-k haben kann, ist zudem eine effiziente dichroitische Zusammenführung bei 2-Lasersystemen möglich, die für die zweitgenannte Variante ausscheidet, da die Wellenlängen sich zum Teil nur sehr wenig unterscheiden.

Grundsätzlich sind beliebige Verteilungen der Schwerpunktwellenlängen in den Beleuchtungspulspaare möglich, solange für jedes Bildpaar eine Korrektur eines Einzelbildes durch Vergleich des anderen Einzelbildes mit einem Einzelbild eines anderen Bildpaares möglich ist und somit eine direkte oder indirekte Referenzierung auf ein anderes, bereits bewegungskorrigiertes oder bewegungskorrigierbares Einzelbild möglich ist. So wäre eine Folge (lambda-0, lambda-1), (lambda-2, lambda-3), (lambda-4, lambda-5), (lambda-6, lambda-7), (lambda-1, lambda-2), (lambda-3, lambda-5), (lambda-4, lambda-7) gleichermaßen geeignet.

Der Weitfeld-SS-OCT ist bevorzugt als vollkohärentes System realisiert. Diese werden auch als Holoskopiesysteme bezeichnet. Das System ist besonders bevorzugt mit einer off-axis Detektionsanordnung realisiert, da dies es bei der Auswertung ermöglicht, den Excess-Noise, sowie den DC-Anteil und den größten Teil des Autokorrelationssignales zu unterdrücken. Dazu wird das von der Probe zurückgestreute Licht unter einem off-axis Winkel mit der Referenzwelle überlagert, zur Interferenz gebracht und detektiert. Es ist dabei wichtig, dass der Kamerasensor in Richtung des off-axis Winkels ein 2- bis 3-fach höheres Sampling realisiert, um alle Ortsfrequenzen der Interferenzen mit der Referenzwelle auflösen zu können. Besonders bevorzugt wird eine 3-Pixel off-axis Detektion verwendet. Dabei weist die Referenzwelle in off-axis Richtung Phasenhübe von etwa 120° pro Pixel auf.

Es wird bevorzugt eine Kamera mit einer Pixelauflösung von deutlich über 1 MPixel verwendet, die bevorzugt technisch als global shutter Kamera ausgeführt ist. Durch diese Ausleseart ist es möglich, zwei Bilder in sehr kurzer Zeit aufzunehmen, was für das hier beschriebene Verfahren wichtig ist. Es gibt aber auch rolling shutter Kameras, die eine ausreichend schnelle Detektion von deutlich über 50 fps bei hinreichender Pixelzahl erreichen und für die beschriebene Detektionsart angewendet werden können.

Für das Verfahren kann die Beleuchtung konventionell mit einer voll kohärenten räumlichen single mode Welle oder alternativ mit einer gestreuten voll kohärenten Multimode-Welle, wie in DE 10 2018 130 396 A1 beschrieben, erfolgen.

Als Anwendung für eine sehr schnelle Volumenbildgebung mit mikroskopischer Auflösung über Messtiefen von vielen Schärfentiefen sind mikroskopische full field OCT-Systeme zu nennen, wie sie beispielsweise in C. Apelian et al., Biomedical Optics Express Vol. 7, Nr. 4, 2016, "Dynamic full field optical coherence tomography: subcellular metabolic contrast revealed in tissues by interferometric signals temporal analysis", beschrieben werden. Da für diese Mikroskopsysteme die Mehrfachstreuung ein sehr wesentliches Problem darstellt, wird für diese Prinzipien besonders bevorzugt eine Streuwellenbeleuchtung und eine Auswertung realisiert, die die Mehrfachstreuung effektiv unterdrückt.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1A: einen Zeitablaufplan für ein Verfahren zur OCT-Abbildung,
- Fig. 1B: ein Blockschaltbild für eine OCT-Vorrichtung,
- Fig. 2A-2C: mögliche Lichtquellen für die Vorrichtung der Fig. 1B
- Fig. 3A-3D: mögliche Strahlgeometrien für eine Beleuchtung in der Vorrichtung der Fig. 1B.

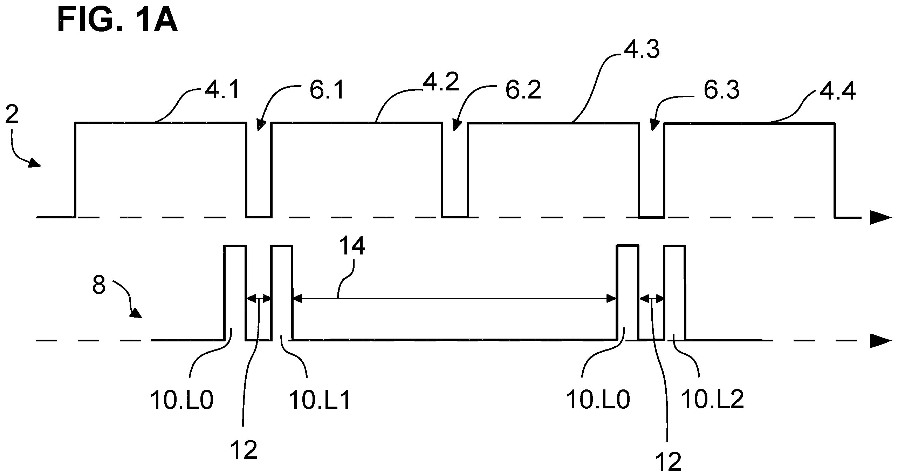

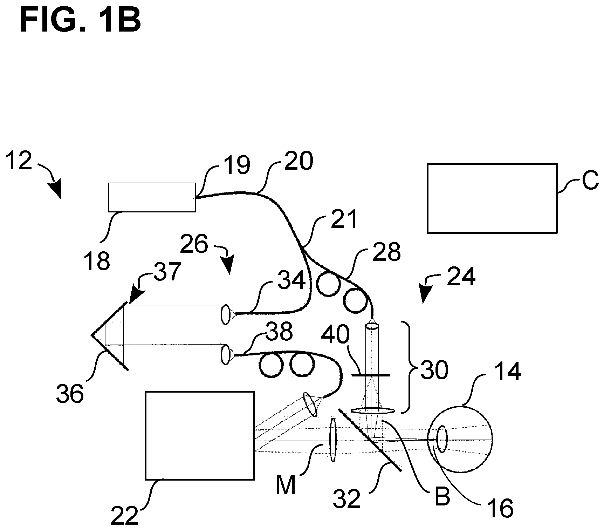

Für eine schnelle SS-OCT-Aufnahme mit einem Bildsensor, dessen Bildwiederholfrequenz, also Mindestbelichtungszeit, eigentlich zu langsam für die gewünschte Abbildungsgeschwindigkeit ist, wird das in Fig. 1 schematisch gezeigte Schema der Belichtung und Auslesung des Detektors eingesetzt. In Fig. 1A ist ein Ablauf der Belichtungsintervalle bei einer üblichen Kamera gezeigt, die hier beispielsweise als global shutter-Sensor ausgebildet ist. Im Ablauf 2 folgen Belichtungsintervalle 4.1, 4.2 usw. aufeinander. Jedes Belichtungsintervall stellt ein Integrationsintervall dar, über welches die Pixel des Sensors elektrische Ladungen aufsammeln, solange sie beleuchtet werden. Die Dauer des Belichtungsintervalls 4.1, 4.2, 4.3 etc. legt die Bildwiederholfrequenz, die sogenannte frame rate, fest. Nach Ablauf der einzelnen Belichtungsintervalle 4.1, 4.2, 4.3 usw. wird jeweils über einen Ausleseintervall 6.1, 6.2 usw. der Sensor ausgelesen, d. h. die Ladungen, die sich in den Pixeln gesammelt haben, werden in ein Ausleseregister übertragen. Dann kann die nächste Integration, d. h. das nächste Belichtungsintervall beginnen. Im Einzelnen heißt dies, dass der Sensor während des Belichtungsintervalls 4.1 in seinen Pixeln Ladungen sammelt, während des Ausleseintervalls 6.1 diese in das Ausleseregister überträgt und währenddessen keine Ladungen akkumulieren kann. Anschließend beginnt ein nächstes Belichtungsintervall 4.2, in dem wieder Ladungen in den Pixeln aufgesammelt werden. Sie werden danach wieder im Ausleseintervall 6.2 ins Ausleseregister übertragen, so dass das nächste Belichtungsintervall 4.3 beginnen kann. Die Dauer der Belichtungsintervalle ist nun so, dass damit die bewegungsbedingten Problematiken an der Probe, beispielsweise am Auge im Falle eines ophthalmologischen Vorderkammer-OCT, nicht mehr tolerabel wären. Es käme insbesondere bei der bevorzugten Holoskopie dazu, dass die erforderliche Phasenstarrheit zwischen den einzelnen Aufnahmen während Durchstimmung des Lasers nicht mehr gegeben wäre.

Es wird deshalb die Belichtung, d. h. die Beleuchtung der Probe derart gestaltet, wie dies der Ablauf 8 in Fig. 1A veranschaulicht. Zum Ende des ersten Belichtungsintervalls 4.1 wird ein kurzer Beleuchtungspuls 10.L0 eingestrahlt, d. h. nur währenddessen fällt im Belichtungsintervall 4.1 Licht auf die Pixel. Zu Beginn des darauf folgenden Belichtungsintervalls 4.2 wird ein Beleuchtungspuls 10.L1 emittiert, der gemäß dem swept-source-Prinzip des SS-OCT bei einer anderen Wellenlänge liegt. Er ist vergleichsweise kurz gegen das Belichtungsintervall 4.2, so dass dieses im Wesentlichen nur Probenlicht aufgrund des Beleuchtungspulses 10.L1 aufsammelt. Der Abstand 12 zwischen den beiden Beleuchtungspulsen 10.L0 und 10.L1 ist durch den Doppelpfeil veranschaulicht und im Wesentlichen nur durch die Dauer der Auslesezeit 6.1 begrenzt. Der Abstand 12 ist so gering, dass in dieser Zeit keine Störungen auftreten, d. h. die erforderliche Phasenstarrheit zwischen dem Bild, das während des Belichtungsintervalls 4.1 durch den Belichtungspuls 10.L0 aufgesammelt wurde, und dem Bild des Belichtungsintervalls 4.2, das vom Beleuchtungspuls 10.L1 herrührt, ist gegeben.

Dieser synchronisierte Ablauf wird nun wiederholt, wobei auf den Beleuchtungspuls 10.L0, der zum Ende des Belichtungsintervalls 4.3 eingestrahlt wird, nun ein Beleuchtungspuls 10.L2 folgt, der eine andere Wellenlänge als der Beleuchtungspuls 10L.1 hat. Der zeitliche Abstand 14 zwischen dem Beleuchtungspuls 10.L1 und dem nächsten Puls ist so groß, dass die erforderliche Phasenstarrheit hier nicht mehr gegeben wäre. Da aber wieder Phasenstarrheit zwischen den aufeinanderfolgend eingestrahlten Beleuchtungspulsen 10.L0 und 10.L2 gegeben ist, kann eine Phasenkorrektur über die Bilder zu dem Beleuchtungspuls 10.L1 (aufgenommen während des Belichtungsintervalls 4.2) und dem Beleuchtungspuls 10.L2 (aufgenommen während des Belichtungsintervalls 10.3) unproblematisch ausgeführt werden.

Somit ist die an und für sich ungenügende frame rate des Bildsensors auf diese Weise ausgeglichen, da Doppelbilder erzeugt werden. Diese Doppelbilder entsprechen jeweils einem Beleuchtungspuls bei einer Referenzwellenlänge, in Fig. 1A der Beleuchtungspuls 10.L0, wobei "L0" die Referenzwellenlänge lamda-0 symbolisiert, und einem Beleuchtungspuls mit sich gemäß der Wellenlängendurchstimmdauer verändernder Wellenlänge, symbolisiert in Fig. 1A durch die Anfügung "L1" bzw. "L2".

Für die Ausführung geeignet ist eine S-65A70 Kamera von Adimec, Eindhoven, NL. Dieser global shutter-Sensor hat eine Auflösung von 65 MPixel und kann mit 70 fps ausgelesen werden. Der Sensor wird auf die längste mögliche Belichtungszeit von 14 ms eingestellt, die noch die volle Framerate realisiert. Nach 14 ms werden die Ladungen, die sich in den Pixeln gesammelt haben, innerhalb von unter 50 µs (frame overhead time) in ein Ausleseregister übertragen, und die Integration des nächsten Frames wird gestartet. Während der Integration des nächsten Frames wird der erste Frame zeitgleich digitalisiert und in die Auswerteeinheit übertragen.

Wird dieses Schema bei der gesamten Kamera mit der gepulsten Beleuchtung geeignet synchronisiert, so dass z. B. die Beleuchtung nur für die letzte Millisekunde des ersten Integrationsintervalls 4.1 aktiviert wird und für die erste Millisekunde des zweiten Integrationsintervalls 4.2, so werden zwei voll aufgelöste Kamerabilder in insgesamt unter 2,05 ms aufgenommen. In so kurzen Zeiten treten keine signifikanten Bewegungsartefakte auf.

Durch die gepulste Betriebsweise können Messgeschwindigkeiten erreicht werden, die deutlich höher sind als die nominellen Frameraten der Kamera. Zudem kann die Lasergrenzwerte zulässigen Spitzenleistungen weiter erhöht werden.

Im SS-OCT-System wird unter Beleuchtung mittels eines SS-Lasers eine definierte Zahl von Bildern zu den Beleuchtungspulsen 10.L1, 10.L2 usw. aufgenommen, während der Laser in seiner k-Zahl (k = 2 pi/lambda) linear und kontinuierlich durchgestimmt wird. Die Durchstimmgeschwindigkeit ist an die für den Tiefenbereich nötigen Anzahl an aufzunehmenden Bildern und an die Framerate der Kamera angepasst.

Für den SS-OCT-Ansatz wird folglich die Durchstimmrate des Lasers kleiner oder gleich der Framerate der Kamera gewählt. Die Framerate der Kamera, die Einstrahlung der Beleuchtungspulse ist gemäß Fig. 1A so an die Durchstimmrate der Kamera angepasst, dass Doppelbilder z. B. gemäß (lambda-0, lambda-1), (lambda-0, lambda-2), (lambda-0, lambda-3), ... (lambda-0, lambda-n) aufgenommen werden. Jedes Doppelbild ist aufgrund der kurzen Aufnahmezeit in sich voll kohärent, die Doppelbilder untereinander können wegen des Abstandes 14 aber Bewegungsartefakte aufweisen, da der Abstand 14 die Bedingung der Phasenstarrheit, die für die Auswertung essenziell ist, nicht erfüllt. Da aber bei jedem Doppelbild immer wieder eine Aufnahme mit der Referenzwellenlänge lambda-0 erfolgt, ist die Phasenlage innerhalb der Doppelbilder fest und kann dadurch mittelbar auch über die Doppelbilder hinweg aufeinander abgestimmt werden. So wird im i-ten Doppelbild die Differenzphase der Bilder zu lambda-0 und zu lambda-i bestimmt und für die weitere Auswertung verwendet. Dadurch werden die durch Bewegungsartefakte entstandenen Phasenfehler kompensiert und die Aufnahmen für lambda-0 bis lambda-i sind wieder hinsichtlich der Auswertung zueinander kohärent.

Das gilt für axiale Bewegungsartefakte. Laterale Bewegungsartefakte werden optional ebenfalls korrigiert, indem die Referenzbilder mit lambda-0 gegeneinander kreuzkorreliert werden. Im Ergebnis erhält man den Verschiebungsvektor zwischen den Aufnahmen und kann damit die lambda-0-Bilder und die dazugehörenden lambda-i-Bilder in ihrer Verschiebung kompensieren.

Die Phasenreferenzierung muss natürlich nicht mit der Wellenlänge lambda-0 erfolgen, sondern kann mit jeder Wellenlänge aus der Wellenlängenfolge oder sogar mit einer zusätzlichen Wellenlänge, die beispielsweise von einem Festfrequenz-Referenzlasers bereitgestellt wird, erfolgen.

Erreicht die Kamera hohe Frameraten, kann es auch ausreichend sein, nur alle 3, 10 oder mehr Wellenlängenbilder eine Referenzmessung mit der festen Wellenlänge durchzuführen. Generell wird die Referenzmessung alle m Bilder (m natürliche Zahl größer Null) ausgeführt.

Die Abstände zwischen den Referenzbildern gleicher Frequenz können auch so groß gewählt werden, dass messbare Bewegungsartefakte/Phasenverschiebungen auftreten. In diesem Fall ist besonders bevorzugt, die Phasenlagen der Referenzbilder 2 pi zu verstetigen (unwraping), die Phasenänderungen für alle Zwischenzeiten zu interpolieren und diese zur Korrektur der unterschiedlichen Wellenlängenbilder zu verwenden.

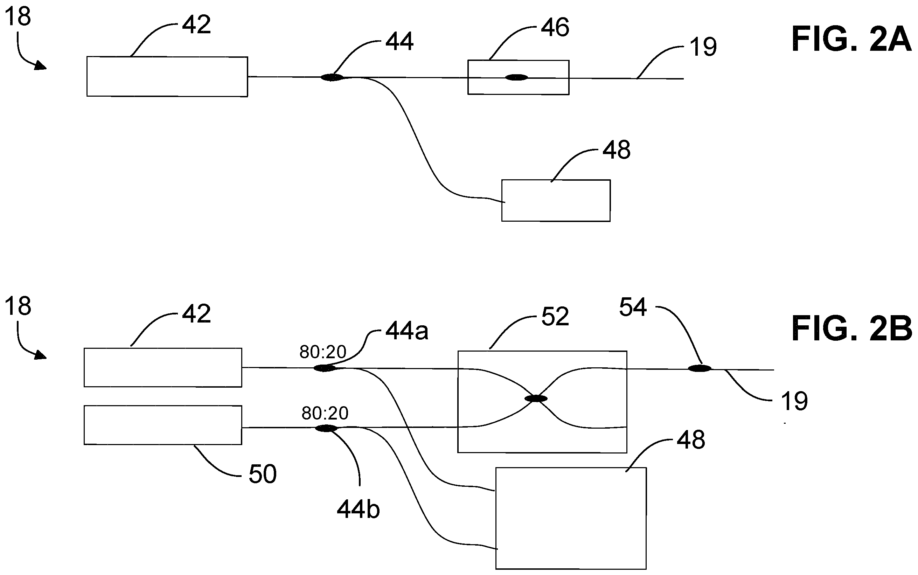

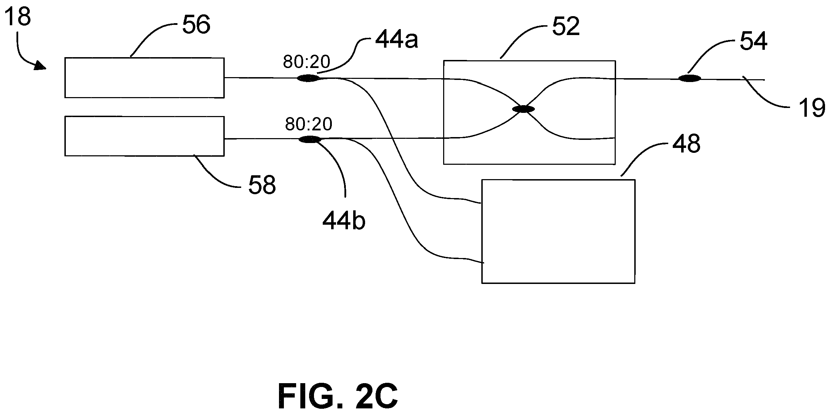

Fig. 1B zeigt schematisch ein OCT 12, das dreidimensionale Bilder eines Auges 14, und zwar insbesondere von der Vorderkammer 16 aufnimmt. Die beschriebene Ausführungsform ist am Beispiel eines faserbasierten swept-source-Systems beschrieben, lässt sich aber auch genauso auf Freistrahlaufbauten übertragen. Quellstrahlung einer hinsichtlich ihrer Wellenlängen durchstimmbaren Strahlungsquelle 18, beispielsweise eines entsprechenden Lasers, wie er nachfolgend exemplarisch anhand der Fig. 2-4 noch erläutert werden wird, wird aus einem Ausgang 19 der Strahlenquelle 18 in eine Faser 20 eingekoppelt. Die Quellstrahlung liegt beispielsweise im infraroten Wellenlängenbereich, wobei in dieser Beschreibung auch dieser Wellenlängenbereich als "Licht" bezeichnet wird. Unter diesem Begriff sei sämtliche Strahlung des elektromagnetischen Spektrums subsummiert, die den optischen Gesetzen genügt. Das Auge 14 wird durch nicht weiter bezeichnete Optiken auf einen Detektor 22 abgebildet, der das anhand Fig. 1A erläuterte Ausleseschema gemäß dem Ablauf 2 hat.

Gemäß der faserbasierten Bauweise mündet die Faser 20 in einen Splitter 21, der die Quellstrahlung in einen Messarm 24 und einen Referenzarm 26 aufteilt. An den Splitter 21 schließt sich im Messarm 24 eine Faser 28 an, und die am Faserende austretende Beleuchtungsstrahlung B wird mittels einer Beleuchtungsoptik 30 hinsichtlich der Beleuchtungsmoden modifiziert - insbesondere ist dazu eine Streuscheibe 40 vorgesehen, deren Wirkung z. B. in DE 10 2018 130 396 A1 beschrieben ist - und dann zu einem Strahlteiler 32 geleitet. Von dort gelangt sie über eine nicht weiter eingezeichnete Frontoptik zur Vorderkammer 16.

Diese Beleuchtungsstrahlung wird in der Vorderkammer 16 aus verschiedenen Tiefen z innerhalb eines Tiefenschärfenbereichs zurückgestreut. Die rückgestreute Strahlung wird als Messstrahlung M aufgesammelt und gelangt zum Detektor 22. Der Strahlteiler 32 trennt damit die Messstrahlung M von der Beleuchtungsstrahlung B. Der Detektor 22 hat eine Ortsauflösung, d. h. er erlaubt eine Auflösung der Intensitätsverteilung über den Strahlquerschnitt gemäß dem Holoskopieprinzip. Der Detektor 22 liegt bevorzugt konjugiert zur Augenpupille. Da aber das Lichtwellenfeld, wenn es in einer Ebene mit Betrag und Phase und bekannt ist, durch eine numerische Propagation in jede andere Ebene umgerechnet werden kann, kann die Detektion auch in anderen Ebenen, z. B. zu einer Ebene in der Vorderkammer 16 erfolgen.

Die vom Splitter 21 in den Referenzstrahlengang 26 abgetrennte Strahlung gelangt in eine Faser 34 und wird über einen Umlenkspiegel 36 sowie eine weitere Faser 38 mit einstellbarer Weglänge schräg auf den Detektor 22 eingestrahlt. Dadurch wird eine Überlagerung von Referenzstrahlung und Messstrahlung M, hier im Wege der sogenannte off-axis-Detektion, erreicht. Die Weglängenanpassung 37 ist im dargestellten Ausführungsbeispiel als Freistrahlengang ausgeführt. Dies ist ebenso optional, wie die Verwendung des Spiegels 36. Dem Stand der Technik sind verschiedene Maßnahmen bekannt, die optische Weglänge eines Strahls einzustellen.

Zum Durchführen der Bilderzeugung weist der OCT 12 ein Steuergerät C auf, das die Wellenlängendurchstimmung der Strahlungsquelle 18 und den Betrieb des Detektors 22 gemäß den anhand Fig. 1A erläuterten Prinzipien synchronisiert, aus den Doppelbildern jeweils die Referenzierung der Phasenbeziehung zum Ausgleich des Abstands 14 vornimmt und entsprechende holoskopische Bilder erzeugt. Letzteres ist im Stand der Technik bekannt.

Der Laser kann aus grundlegenden physikalischen Gründen, um einen für die Messung notwendigen zeitlichen Kohärenzgrad zu erreichen, während der Messung nicht moduliert werden. Daher wird das emittierte Licht des Lasers, wenn nötig, mit einem Verstärker (z. B. SOA) verstärkt und dann durch einen schnellen optischen Switch geschaltet. Wird ein SOA verwendet, so kann dieser in die optische Schaltung mit einbezogen werden.

Für das beschriebene Verfahren und die Vorrichtung sind Swept-Source-Laserquellen 18 bevorzugt, deren Wellenlängencharakteristik so vorhersagbar ist, dass die Pulssteuerung ohne online-Überwachung der Laserwellenlänge, d. h. ohne Wellenlängenmessanordnung (k-clock), erfolgen kann. Wenn das nicht möglich ist, wird eine k-clock-Anordnung verwendet, wie sie im Stand der Technik bekannt ist, um die Synchronisation optischer Schalter zu steuern.

In J. Kühn et al., Optics Express Vol. 15, Nr. 12, 2007, "Real-time dual-wavelength digital holographic microscopy with a single hologram acquisition", ist ein Verfahren der digitalen Mehrfrequenz-Holographie beschrieben, bei dem in einem Kamerabild zwei Wellenlängen lambda-0 und lambda-n gleichzeitig aufgenommen werden. Die Referenzwellen der beiden Wellenlängen werden dabei aus unterschiedlichen Richtungen so eingestrahlt, so dass sie bei der Auswertung durch eine geeignete laterale Filterung voneinander getrennt werden können. Der Vorteil dieser Beleuchtungsanordnung ist es, dass die Referenzaufnahme exakt zur gleichen Zeit wie die Messaufnahme realisiert wird, wodurch Bewegungsartefakte noch besser unterdrückt werden. Dies kann nun mit der rolling shutter Kamera realisiert werden, und das schnelle Schalten der Laser für die Realisierung der Integrationszeit wird durch die Kameraintegration erreicht. Somit können die Laserquellen einfacher realisiert werden. Ein Nachteil dieser Anordnung ist es, dass man in der Richtung senkrecht zur off-axis-Detektion die Aufnahme 2-fach dichter abtasten muss, um die Filterung zu realisieren. Dadurch verliert man effektiv die Hälfte der Kamerapixel. Außerdem wird durch die zwei Referenzwellen das SNR um 3 dB reduziert. Diese Ausführungsform wird daher bevorzugt für die Messung sehr dynamischer Objekte verwendet.

Wird das beschriebene Verfahren mit einer Streuwellen-Beleuchtung kombiniert, so erfolgt die Auswertung leicht abgewandelt. In diesem Fall ist es nicht ausreichend, die Phase der Referenzmessung abzuziehen, da durch die Propagation der gestreuten Welle in die Probe Phasen und Amplitudenänderungen entstehen, die nicht ortsvariant sind und sich somit bei einer Verschiebung des Objektes in Bezug auf das Messgerät verändern. Die Ortsfrequenzen die durch die gestreute Specklebeleuchtung eingebracht werden, mischen mit dem nicht limitierten Ortsfrequenzspektrum des zu messenden Objektes. Durch die Filterung, die anschließend durch eine Detektionsblende realisiert wird, wird dieses Mischfrequenzspektrum gefiltert. Eine eindeutige "Entmischung" des übertragenen, gefilterten und detektierten Ortsfrequenzspektrums ist dann nicht mehr aus einem einzigen Wellenlängen-Bildstapel möglich. Daher wird für die weitere Diskussion zwischen zwei Auswertemodi unterschieden, einem einfachen Modus, bei dem genau ein Wellenlängen-Bildstapel ausgewertet wird und einem erweiterten Auswertemodus, bei dem mehrere, bevorzugt etwa 10 bis 40 Wellenlängen-Bildstapel kohärent verrechnet werden.

Bei der einfachen Ausführungsform wird in der Auswertung nicht versucht, das Probenortsfrequenzspektrum, das mit dem gestreuten Beleuchtungswinkelspektrum gemischt wurde zu entmischen. Es wird vielmehr im Endbild die Mischung von Detektionsspecklen, die durch die Rückstreuung im Objekt entstehen, mit den Beleuchtungsspecklen dargestellt. Damit diese Methode der Rekonstruktion keine Artefakte verursacht, ist wichtig, dass sich die Beleuchtungsspeckle während der Aufnahme aller Bilder, die kohärent verrechnet werden sollen, um deutlich weniger als ein Specklekorn untereinander und gegenüber dem Objekt lateral und axial unterscheiden. Für die einfache Auswertung ist es daher vorgesehen, dass die Streuscheibe in die mittlere Objektebene abgebildet wird, damit auch die Specklemuster der verschiedenen Wellenlängen bis auf eine für das Verfahren charakteristische wellenlängenabhängige Propagationsphase annähern gleich sind. Der damit erreichbare Tiefenbereich skaliert mit lambda²/(nA²*delta-lambda).

Außerdem sollten die Bilder möglichst schnell aufgenommen werden. Wenn die Aufnahmen sich nur um einen festen Punkt verschieben, wie es z. B. bei Aufnahmen einer menschlichen Retina der Fall ist, die auf ein Fixierlicht fixiert wird, so ist es alternativ möglich, die Beleuchtungsapertur so klein zu wählen, dass die entstehenden Beleuchtungsspeckle größer als die Verschiebungsamplitude sind.

Eine dritte Möglichkeit besteht darin, die Beleuchtungsapertur deutlich kleiner (<3-mal) als die Detektionsapertur zu wählen. In diesem Fall entstehen nur geringfügige Artefakte, die sich in einem höheren, aber noch akzeptablen Bildrauschen zeigen. In diesem Fall ist die Beleuchtungswelle aber meist so stark in ihrem Etendue eingeschränkt, dass die nutzbaren Lichtgrenzwerte nur geringfügig größer als von denen einer single-mode Beleuchtung sind.

Treten schnelle Bewegungsartefakte mit großer Amplitude und hohen Anforderungen an die zu erzielende laterale Auflösung und Bildqualität auf oder sollen sehr tiefe Objektstrukturen vermessen werden, die tiefer als der im vorigen Abschnitt beschriebene Tiefenbereich sind, so erfolgt bevorzugt eine erweiterte Auswertung. Es werden mehrere unabhängige Wellenlängen-Bildstapel aufgenommen und kohärent verrechnet. Es gibt drei Varianten dieser erweiterten Auswertung, die auch unterschiedliche Eigenschaften aufweisen. Bei einigen dieser Varianten können zusätzlich die laterale Auflösung verdoppelt und Mehrfachstreuung in den Aufnahmen unterdrückt werden.

Für die im Folgenden beschriebene erweiterte Auswertung ist es bevorzugt, die Streuscheibe 39 in die Objektebene abzubilden, es ist aber auch für einige der Varianten möglich, die Streuscheibe z. B. pupillennah zu stellen. Das ist besonders in linsenlosen Ausgestaltungsformen, wie sie im Stand der Technik bekannt sind, wichtig. Das spezifische Problem dieser Art der Auswertung ist, dass alle Aufnahmen kohärent miteinander verrechnet werden müssen. Durch die Aufnahme mehrerer Bildstapel auch mit Kameras hoher lateraler Auflösung wird, wie erläutert werden wird, eine phasenstarre Bildaufnahme auch über mehrere Sekunden realisiert.

Fig. 2 zeigt eine Lichtquelle 19 mit nur einem Laser 42 für die nachfolgende erste und zweite Beleuchtungsvariante.

Die erste Beleuchtungsvariante wurde bereits erwähnt. Es gibt nur eine durchgestimmte Laserquelle 42, mit Durchstimmraten in der Größenordnung der Framerate der Kamera 22. Durch die schnelle Schaltung 40 der Quelle 19 synchronisiert mit der Aufnahme der Kamera 22 werden die Doppelbilder mit den Wellenlängen (lambda-0, lambda-n) geeignet ausgeschnitten und detektiert. In diesem Fall werden nur eine Quelle 42, ein optionaler Splitter 44 für eine optionale k-clock 48 (s. o.) und ein schneller Schalter 46 benötigt. Nachteilig an dieser Ausführung ist, dass der zeitliche Abstand zwischen lambda-0 und lambda-n nicht beliebig kurz gewählt werden kann, da er durch die Durchstimmzeit des Lasers 42 begrenzt wird. Außerdem ist bei dieser Art der Detektion die Integrationszeit für ein einzelnes Bild lambda-i eingeschränkt, da der Laser 42 kontinuierlich durchstimmt wird und nur eine gewisse Wellenlängendrift während der Integrationszeit toleriert werden kann.

Die zweite Beleuchtungsvariante unterscheidet sich von der ersten nur durch eine andere Laseransteuerung. In diesem Fall wird der Laser 42 nicht kontinuierlich durchgestimmt, sondern zwischen festen Wellenlängen umgeschaltet. Auf jeder neuen Wellenlänge benötigt der Laser eine bestimmte Einschwingzeit von etwa 1 bis 100 ms, um wieder in einen stabilen Langkohärenz-Betrieb zu kommen. In diesem Fall kann das Integrationsintervall 4.1 etc. deutlich länger gewählt, sodass es nur durch die Bewegungsartefakte limitiert wird. Der minimale zeitliche Abstand innerhalb des Doppelbildes entspricht dabei aber der Einschwingzeit. Diese Lösung ist daher besonders bevorzugt für Lasersysteme, die in unter zirka 10 ms in einen stabilen Betrieb einschwingen. Während der Einschwingzeit wird der Laser durch den Schalter 46 geblockt, um die Kamerasignale nicht zu verfälschen.

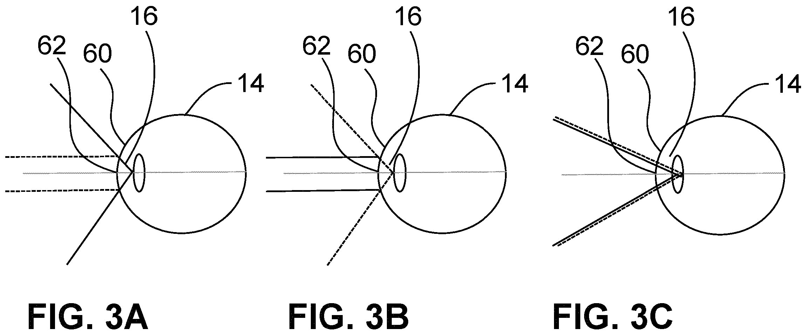

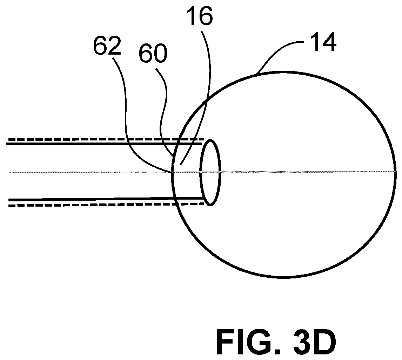

Eine dritte Beleuchtungsvariante gemäß Fig. 3 arbeitet mit zwei Lasern 42, 50, einem Festfrequenzlaser 50 mit der Wellenlänge lambda-0 und einem zweiten durchstimmbaren Laser 42. Der durchstimmbare Laser 42 wird wie bei Fig. 2 entweder zwischen diskreten Wellenlängen lambda-i umgeschaltet oder langsam kontinuierlich durchgestimmt. Langsam meint z. B. in diesem Fall, dass wenn 400 Wellenlängen lambda-i verwendet werden, der Laser 42 in zirka 11 Sekunden einmal durchgestimmt wird, um in dieser Zeit mit der Kamera 400 Doppelbilder lambda-0, lambda-i aufnehmen zu können. In diesem Fall übernimmt ein schneller optischer Umschalter 52 die Zusammenführung der beiden Laserstrahlungen und zusätzlich die schnelle optische Schaltung. Diese Variante ist technisch die aufwendigste, erlaubt aber sehr schnelle Doppelbilder und lässt lange Integrationsintervalle zu. In dieser Konstellation ist es besonders bevorzugt, wenn ein SOA/optischer Nachverstärker 54 hinter dem Umschalter 52 realisiert wird, um beide Laser 42, 50 verstärken zu können. Diese Variante kann auch bevorzugt mit der beschriebenen zeitgleichen 2-Referenzaufnahme kombiniert werden. In diesem Fall wird ein Intensitätsteiler anstelle des optischen Umschalters 52 eingesetzt und die Belichtung durch das Integrationsintervall der Kamera realisiert. Die Referenzwellen für die k-clock 48 werden vor der Zusammenführung im Teiler 44a, 44b abgeteilt. Dadurch beschränkt sich der Mehraufwand auf die Realisierung des zweiten Festfrequenzlasers 50. Da für die meisten OCT-Aufnahmen aber nur Kohärenzlängen im 10 mm Bereich benötigt werden, können technisch einfache Referenzlaserdioden eingesetzt werden.

Nutzt man das dargestellte Aufnahmeverfahren für Aufnahmen der Cornea und der Vorderkammer 16 des menschlichen Auges 14, so kann man durch bestimmte Einstrahlgeometrien die Anzahl der aufzunehmenden Bilder für bestimmte Anwendungen minimieren. Es sollen im Folgenden verschiedene Einstrahlgeometrien mit ihren Vor- und Nachteilen beschrieben werden.

Der Vorteil des hier beschriebenen OCT-Systems ist eine mikroskopische Auflösung im Mikrometerbereich über Gewebetiefen von einigen Millimetern. Es ist durch die Parametrisierung möglich, die Schärfentiefe des Systems und die Tiefenauflösung, die durch die Kohärenzmessung erreicht wird, unabhängig voneinander einzustellen. Es ist aber für die voll-kohärente 3D-Rekonstruktion wichtig, dass die erreichte Kohärenz-Tiefenauflösung besser oder gleich der Schärfentiefe des Systems gewählt wird.

Soll die mikroskopische Auflösung nur im Corneagewebe 60 erreicht werden, so ist bevorzugt die Beleuchtungsstrahlung so einzustrahlen, dass eine gewölbte zero delay-Fläche in der Nähe der Corneavorderseite entsteht. Die Form und Lage des zero delays wird gleichermaßen von Beleuchtungs- und rückgestreuter Detektionswelle beeinflusst. Um eine zero delay-Fläche auf der Corneavorderseite zu erzeugen, kann man das Fermat-Prinzip anwenden. Stellt man sich die Corneavorderseite verspiegelt vor und realisiert die Beleuchtung so, dass sie durch die Corneareflexion in die Detektion abgebildet wird, so ist das zero delay durch eine Längeneinstellung des Referenzarmes auf die Corneaoberfläche eingestellt. Es sind dafür 3 Geometrien denkbar, wie sie in Fig. 4A-C gezeigt sind. Es können aber natürlich auch Mischformen realisiert werden. Die Detektion erfolgt mit einer bestimmten numerischen Apertur. In Fig. 4A-C sind werden aber vereinfacht nur die Hauptstrahlen dargestellt. Die Figur zeigt Strahlgeometrien für zero delay auf der Corneavorderseite 61 (Beleuchtung B gestrichelt, Detektion M durchgestrichen gezeichnet), der mittlere Krümmungsradius der Cornea 60 ist 8 mm. In Fig. 4A zielt die Detektion etwa 4 mm hinter den Corneascheitel 62. In Fig. 4B zielt die Beleuchtung etwa 4 mm hinter den Corneascheitel 62. In Fig. 4C zielen beide etwa 8 mm hinter den Corneascheitel 62.

Der Vorteil einer der Strahlführungen gemäß Fig. 4 ist es, dass man mit einem messbaren Tiefenbereich von etwa 700 µm im Gewebe oder etwa 1 mm in Luft die gesamte Cornea 60 mit hoher Auflösung aufnehmen kann. Nachteilig an dieser Anordnung ist es, dass der sehr helle Corneavorderseitenreflex die Dynamik der Aufnahme übersteuern kann. Außerdem benötigt man größere Optikdurchmesser, um die Strahlführung zu erreichen.

Fig. 5 zeigt eine dahingehende verbesserte Beleuchtung mit parallelem Bündel und telezentrische Detektion. Sie eignet sich besser für Aufnahmen der gesamten Vorderkammer und für Aufnahmen von kleineren Subfeldern. Außerdem können für z. B. Aufnahmen vom Schlemm-Kanal die Einstrahlwinkel flexibler gewählt werden, um die Bildgebung durch möglichst geringe Wege in der Sklera zu verbessern. Dazu ist es besonders bevorzugt, wenn die Messeinrichtung neben einer x, y und z Justage über einen bei ophthalmologischen Geräten genutzten Kreuztisch auch über eine Schwenkvorrichtung (evtl. Neigevorrichtung) verfügt, wie sie bei Funduskameras und Spaltlampen bekannt sind.

Es ist bevorzugt, den optischen Schalter vor der Aufteilung von Signalbeleuchtungswelle und Referenzwelle anzuordnen, um auch die Referenzwelle zu schalten und somit den DC-Anteil in den Kameraaufnahmen zu minimieren.

Die Verfahren, die im Stand der Technik für die Bewegungskompensation entwickelt wurden, versuchen die Bewegungen nur innerhalb eines Wellenlängen-Bilderstapels zu kompensieren. Dazu wird bei einem Wellenlängen-Scan versucht, zwischen Signaländerungen, die durch die Änderung der Wellenlänge und Signaländerungen, die durch Bewegungsartefakte verursacht werden zu unterscheiden und dann die Bewegungsartefakte zu kompensieren. Dies hat drei applikative Einschränkungen, die eine Übertragung auf die hier beschriebene Fragestellung nicht möglich machen. Das Verfahren eignet sich nur dazu, geringfügige Bewegungen innerhalb der Aufnahme des Bildstapels zu korrigieren. So kann die Kohärenzzeit von einigen Millisekunden um etwa den Faktor 10 verlängert werden. Um auch die dafür notwendigen extrem schnellen Frameraten der Kamera zu erreichen, müssen Hochgeschwindigkeitskameras eingesetzt werden, die eine reduzierte Auflösung nur bis etwa 1 MPixel haben. Außerdem müssen in den aufzunehmenden Objekten ausreichend laterale als auch axiale Strukturen vorhanden sein, um das Schätzen der Bewegungsartefakte zu ermöglichen. Das ist besonders aufgrund der geringeren Pixelzahl schwieriger als für hochauflösende Kamerasensoren, die hier verwendet werden. Zusammengefasst schätzen die Verfahren aus dem Stand der Technik die Bewegungen aus schnellen Wellenlängenbildsequenzen, während das hier vorgestellte Verfahren bzw. die Vorrichtung die Schätzung der Bewegungen auch aus Einzelbildern vornimmt.

Durch die Bewegungsartefakte, die sowohl innerhalb der Aufnahme der Bildstapel als auch zwischen den Bildstapeln auftreten, haben die Bilder gleicher Wellenlänge in den unterschiedlichen Stapeln eine unterschiedliche zufällige Verschiebung des Beleuchtungsspecklemusters in Bezug auf das zu messende Objekt, die größer als ein Specklekorn sein sollte.

Im Folgenden soll als erstes die besonders bevorzugte erste Untervariante der erweiterten Auswertung beschrieben werden und dann nur die Unterschiede der beiden anderen möglichen Untervarianten diskutiert werden.

Für die erweiterte Auswertung werden in einem ersten Schritt die Bewegungen zwischen den Bildpaaren durch einen Vergleich der Aufnahme mit der Referenzwellenlänge geschätzt und damit die Aufnahme der zeitnah ohne Bewegungen aufgezeichneten zweiten Wellenlänge digital korrigiert, sodass danach alle Aufnahmen der mehreren Bildstapel zueinander kohärent sind.

Als zweiter Schritt werden die Bilder der verschiedenen Stapel neu angeordnet und dabei aus allen Bildern der jeweils gleichen Wellenlänge ein auflösungsgesteigertes Bild rekonstruiert, das auch je nach Anzahl der verrechneten Stapel eine unterdrückte Mehrfachstreuung aufweist. Dazu werden die berechneten Signalfeldstärken der verschiedenen Aufnahmen einfach addiert. Durch die Phasenkorrektur im ersten Schritt werden dabei die einfach gestreuten Signalanteile konstruktiv überlagert, während die mehrfach gestreuten Signale Phasen-unkorreliert überlagert werden und sich damit teilweise aus den Signalen herausmitteln.

Der dritte Schritt ist dann wieder identisch zur bekannten holoskopischen Rekonstruktion, es werden die so erhaltenen auflösungsgesteigerten Bilder der verschiedenen Wellenlängen durch eine Fouriertransformation zu einem tiefenaufgelösten Endbild verrechnet. Es gibt im Stand der Technik auch kompliziertere 3D-Auswerteverfahren, die alternativ genutzt werden können, hier aber im Folgenden vereinfacht als Fouriertransformation bezeichnet werden.

Für den besonders wichtigen ersten Schritt der Auswertung wird aus jedem aufgenommenen Bildpaar das Referenz-Wellenlängenbild extrahiert und mit einem festen Vergleichsbild, z. B. dem ersten Referenzwellenlängenbild verglichen. Die beiden Bilder werden lateral gegeneinander kreuzkorreliert und dann die laterale Verschiebung korrigiert. Die gleiche Verschiebung wird auch in dem zugehörigen zweiten Wellenlängenbild korrigiert. Als nächster Schritt werden die Feldstärken des ersten und n-ten Referenzbildes miteinander verglichen. Die Phasenwerte werden aus drei physikalisch unterschiedlichen Gründen voneinander abweichen.

Zum Ersten, weil sich die beiden Referenzbilder durch axiale Bewegungen in der absoluten Phase geändert haben können. Diese Änderungen können zu einer konstanten globalen Phase führen, bei den meisten Anwendungen werden aber auch höhere laterale Ordnungen wie z. B. Kippwinkel (Phasenrampen) und Defokus (quadratische laterale Phasenverteilungen) auftreten und müssen korrigiert werden. Diese Phasenfunktionen sind aber lokal sehr stark korreliert, sodass die Phasenveränderung nicht für jeden Pixel individuell geschätzt werden muss, sondern eine parametrisierte Phasenfunktion mit einigen wenigen, bis einigen 10 Parametern angefittet werden kann.

Als zweites wird durch die gestreute Beleuchtungswelle mit ihrem typischen Specklemuster in jedem Specklekorn eine zufällige Anfangsphase auftreten. Auch wird die Anregungsfeldstärkebetrag durch diese Speckle über das Objekt lateral und axial variieren. Diese Speckleamplituden und -phasen werden als bekannt vorausgesetzt. Sie können aus dem Design der Streuscheibe abgeleitet werden oder können durch eine Kalibriermessung mit einem Spiegel als Objekt in einer Ebene vermessen werden und von dort mit den Methoden der digitalen Propagation auf alle Punkte des beleuchteten Objektes übertragen/umgerechnet werden. Der Spiegel/Hohlspiegel wird dabei so ausgelegt, dass er die Ausgangspupille der Streubeleuchtung in die Eingangspupille der Detektionsoptik abbildet. Ist der Strahlengang objektseitig telezentrisch, so werden Planspiegel verwendet, ansonsten Hohlspiegel. Die gemessene Feldverteilung der gestreuten Anregungswelle wird im Folgenden als Kalibriermessung bezeichnet und wird für jede Wellenlänge individuell aufgenommen. Das gemessene Signalfeldstärkebild wird durch die so bekannte Anregungsfeldstärke/Kalibriermessung komplex geteilt, um diesen Einfluss der Specklebeleuchtung zu kompensieren. Beide Größen werden in der konjugierten Bildebene/Zerodelay bestimmt und verrechnet. Da bei dieser Teilung doppelte Ortsfrequenzen entstehen können, werden Signalfeldstärkebild und Kalibrierbild vor dieser Operation auf doppelte Pixeldichte interpoliert.

Der dritte Effekt kommt durch eine Wechselwirkung der Beleuchtungsortsfrequenzen mit den Ortsfrequenzen des Rückstreukoeffizienten des Objektes zustande. Damit verändert sich der Amplituden- und Phasenwert in einem Objektpunkt abhängig vom Gradienten der Anregungsfeldstärke in diesem Punkt, der für jede Lage des Beleuchtungsspecklemusters in Bezug auf die Probe unterschiedlich sein wird.

Wird z. B. mit einem Beleuchtungsspecklemuster beleuchtet, dass die gleiche Winkelapertur aufweist wie die Detektion, so sind durch die strukturierte Specklebeleuchtung die doppelten Ortsfrequenzwerte des Objektes zugänglich, die sich allein aus der beugungsbegrenzten Detektion berechnen würden. Es wird aber von der Detektion nur das einfache Ortsfrequenzspektrum übertragen. Aus diesem Grund wird in diesem Beispiel nur ein Viertel der Ortsfrequenzinformationen in einem Bild übertragen und es ist prinzipiell aus einer Einzelaufnahme nicht möglich diesen Phasen- und Amplitudeneffekt zu bestimmen. Bei der späteren Verrechnung von ausreichend vielen Bildern der gleichen Wellenlänge aber unterschiedlicher Beleuchtungsspeckleverschiebungen können dann alle Ortsfrequenzen bis zur doppelten Detektionsgrenzfrequenz eindeutig rekonstruiert werden. Dafür ist aber die kohärente Verrechnung zwingend notwendig, für die man vorher die Verschiebephasen bestimmt und korrigiert haben muss. Insofern kann dieser Effekt hier bei der Bestimmung der Phasenverschiebung der Einzelbilder nicht kompensiert werden und man muss mit seinen Auswirkungen umgehen. Nach der Korrektur der detektierten Signalfeldstärkebilder durch die Anregungsfeldstärken führt der so beschriebene Effekt in diesem Beispiel zu einer Phasendekorrelation von zirka 75%. Um dennoch die Phasenlage der Referenzbilder gegeneinander schätzen zu können muss lateral über mehrere (viele) Pixel gemittelt werden. Die globale Phase kann durch Mitteln aller Pixelphasen ermittelt werden. Für die praktische Anwendung ist aber der beschriebene Ansatz mit dem Anfitten einer parametrisierten Phasenfunktion mit deutlich weniger Parametern als Detektionspixel der deutlich bevorzugte Weg. Hat man die Phasen auf diese Art und Weise bestimmt, so werden die Phasen von der Referenzbildwellenlänge Phi-ref auf die der Messbildwellenlänge Phi-mess umgerechnet (Phi-mess = Phi-ref * lamda-mess / lambda-ref) und in diesen Messbildern mit variabler Wellenlänge korrigiert.

Die beiden letzten Schritte der Auswertung, das kohärente Summieren der Feldstärken aller Bilder gleicher Wellenlänge nach der beschriebenen Korrektur und die anschließende Fouriertransformation über die verschiedenen Wellenlängen kann aufgrund der Linearität der Fouriertransformation auch in der umgekehrten Reihenfolge erfolgen. Dazu wird jeder korrigierte Bildstapel einzeln Fourier transformiert und dann die komplexen Ergebnisfelder kohärent addiert.

Die erste Untervariante der erweiterten Auswertung hat damit zusammengefasst folgende Eigenschaften: Die Streuscheibe muss nicht zwingend in die Objektebene abgebildet werden, sodass sich diese Variante auch für linsenlose Systeme eignet. Die Anregungsspeckleverteilung muss aber durch die Kalibriermessung bekannt sein. Das Ergebnisbild wird je nach Wahl der Beleuchtungsapertur eine doppelte laterale Auflösung aufweisen. Je nach Anzahl rekonstruierten Bildstapel wird die Mehrfachstreuung im Ergebnisbild unterdrückt, da sie sich teilweise herausmittelt. Die Ergebnisbilder werden aber voll verspeckelt sein. Um die Bilder teilweise zu entspeckeln, können 4 Pixel im Ergebnisbild inkohärent gemittelt werden, wodurch sich aber die laterale Auflösung auf die normale Detektionsauflösung reduziert.

In der zweiten Untervariante der erweiterten Auswertung werden die Signalbilder auch erst durch die Kalibriermessung geteilt, dann die lateralen Verschiebungen und die axialen Phasen durch Bewegungsartefakte kompensiert. Es wird dann aber für jeden Bildstapel erst die Fouriertransformation über die Wellenlängen berechnet. Die daraus resultierenden tiefenaufgelösten Bilder der unterschiedlichen Bildstapel werden dann aber inkohärent addiert, d. h. es werden die Beträge der Funktion berechnet und addiert. Als Ergebnis erhält man ein Endbild mit normaler durch die Detektion beugungsbegrenzter lateraler Auflösung in dem die Mehrfachstreuung nicht unterdrückt ist, das aber je nach Anzahl der verrechneten Stapel auch vollständig entspeckelt sein kann. Diese Entspeckelung bezieht sich sowohl auf die einfach gestreuten Signalanteile als auch auf die mehrfach gestreuten Signalanteile, sodass die Endbilder sehr glatt/rauscharm aussehen werden. Es ist dann durch eine Erhöhung des dargestellten Bildkontrastes möglich, die in dem Bild sichtbare Mehrfachstreuung virtuell zu unterdrücken. Für diese Variante muss die Streuscheibe nicht in die Probe abgebildet werden, wodurch sie sich auch für linsenlose Systeme eignet.

In der dritten Untervariante der erweiterten Auswertung wird das Signalbild nicht durch die Kalibriermessung geteilt. Es werden aber die lateralen Verschiebungen und die aufgrund der Bewegung auftretenden axialen Phasenänderungen wie in den anderen Untervarianten beschrieben korrigiert. Wie bei der ersten Untervariante werden dann die Bilder erst kohärent addiert und dann die Fouriertransformation über die Wellenlängen berechnet oder auch in umgekehrter Reihenfolge. Diese Variante der Auswertung funktioniert nur, wenn wie bei der einfachen Auswertung die Streuscheibe scharf in die Probe abgebildet und der dargestellte maximale Tiefenbereich eingehalten wird. In diesem Fall sind die Anfangsphasen der gestreuten Anregungswelle im Zerodelay nicht bekannt, sie sind aber in allen Wellenlängen annähern gleich und heben sich dadurch bei der Auswertung heraus. Daher werden bei dieser Variante die einfachgestreuten Signalanteile konstruktiv und die mehrfach gestreuten Signalanteile unkorreliert überlagert, wodurch die Bilder eine unterdrückte Mehrfachstreuung zeigen werden. Da aber die Intensitätsschwankungen der gestreuten Beleuchtungswelle nicht korrigiert werden, die auch die Übertragung der Ortsfrequenzen beeinflussen, werden sich die höheren Beugungsordnungen herausheben und das Ergebnisbild nur die einfache durch die Detektion beugungsbegrenzte Auflösung aufweisen. Die erhaltenen Ergebnisbilder sind aber zumindest teilweise entspeckelt. Die Auswertung nach der dritten Untervariante ist auch in DE 10 2018 130 396 A1 in Grundzügen beschrieben.

Ein Merkmal der beschriebenen Anordnung ist der sehr hohe Parallelisierungsgrad mit vielen Megapixeln Auflösung. Dadurch ist die Anregungsintensität pro Pixel sehr gering, sodass entweder sehr lange Integrationszeiten folgen oder sehr leistungsstarke Laser eingesetzt werden müssen. Im Folgenden sollen daher spezielle Ausgestaltungen des Lichtquellensystems beschrieben werden, die für diesen Anwendungsbereich bevorzugt sind.

Die dargestellten Anordnungen zeigen nur die Aspekte der durchlaufenen absoluten Strahlwege und keine Abbildungsleistungen und Bildfeldlagen. Die Korrektur so großer Bildfelder bis zirka 12 mm für numerische Aperturen von 0,2 und größer bei gleichzeitig beugungsbegrenzter Auflösung sind optisch sehr schwer zu erreichen, speziell auch für Bildfeldebenen die konvex gekrümmt sind. Ein großer Vorteil der voll kohärenten Weitfeld-OCT ist es aber, dass bekannte Bildfelder bei der Auswertung aus den Daten herausgerechnet werden können, in dem die detektierten Lichtwellenfelder numerisch bis zur Pupille propagiert werden, dort die Phasenfunktion der Aberrationen korrigiert und die Welle anschließend in die Feldebene numerisch zurückpropagiert wird. Dieses Vorgehen ist im Stand der Technik bekannt. Hier ist darüber hinaus besonders bevorzugt, dass die Optik auf maximale Schärfe in den Rändern des Bildfeldes zu Lasten der Korrektur in der Mitte des Bildfeldes (speziell bei der Bildfeldwölbung) korrigiert wird, um die Randeffekte/Überlappbereich bei der numerischen Propagation zu minimieren.

## Patentansprüche

1. Weitfeld-Swept-Source-OCT-Verfahren zur Abbildung eines bewegten Objekts (14), insbesondere der Vorderkammer (16) des menschlichen Auges, wobei das Verfahren folgende Schritte aufweist:
- Bereitstellen von in der Wellenlänge durchgestimmter Beleuchtungsstrahlung (B), die einzelne Beleuchtungspulse (10.L0, 10.L1) unterschiedlicher Schwerpunktwellenlänge aufweist, wobei die Beleuchtungspulse (10.L0, 10,L1, 10.L2) als Serie von Beleuchtungspulspaaren (10.L0, 10L1; 10.L0, 10.L2) bereitgestellt werden, die jeweils aus einem ersten Beleuchtungspuls (10.L0) und einem zweiten Beleuchtungspuls (10.L1, 10.L2) bestehen, wobei in den Beleuchtungspulspaaren (10.L0, 10L1; 10.L0, 10.L2) sich die Schwerpunktwellenlänge des ersten Beleuchtungspulses (10.L0) von der Schwerpunktwellenlänge des zweiten Beleuchtungspulses (10L1, 10.L2) unterscheidet in mehreren der Beleuchtungspulspaare (10.L0, 10L1; 10.L0, 10.L2) mind. eine Schwerpunktwellenlänge eines der Beleuchtungspulse (10.L0) eines vorhergehenden der Beleuchtungspulspaare (10.L0, 10.L1; 10.L0, 10.L2) wiederholt wird,
- Beleuchten des Objekts (14) mit der Beleuchtungsstrahlung (B) und Abbilden des beleuchteten Objekt (14) auf einen 2D-Detektor (22),
- Betreiben des Detektors (22) gemäß einem Bildaufnahmezyklus umfassend eine Abfolge (2) von Belichtungsintervallen (4.1, 4.2, 4.3, 4.4) und Ausleseintervallen (6.1, 6.2, 6.3),
- Synchronisieren des Abgebens der Beleuchtungspulse (10.L0, 10,L1, 10.L.2) und des Betreiben des Detektor (22) derart, dass die Beleuchtungspulspaare (10.L0, 10L1; 10.L0, 10.L2) um jedes zweite (6.1, 6.3) der Ausleseintervalle (6.1, 6.2, 6.3) der Abfolge (2) herum gruppiert sind und für jedes Beleuchtungspulspaar (10.L0, 10L1; 10.L0, 10.L2) der erste Beleuchtungspuls (10.L0) während eines letzten Drittels eines der Belichtungsintervalle (4.1, 4.2, 4.3, 4.4) und der zweite Beleuchtungspuls (10L1; 10.L2) während eines ersten Drittels des folgenden der Belichtungsintervalle (4.1, 4.2, 4.3, 4.4) abgegeben wird, und
- Auslesen von Bilddaten jedes Belichtungsintervall (4.1, 4.2, 4.3, 4.4) des Detektors (22) und Zuordnen der Bilddaten zu den Schwerpunktwellenlängen des im jeweiligen Belichtungsintervall abgegebenen Beleuchtungspulses (10.L0, 10,L1, 10.L.2), wobei Bildpaare bestehend aus Einzelbildern entsprechend den Beleuchtungspulspaaren (10.L0, 10L1; 10.L0, 10.L2) erzeugt werden,
- Ermitteln von Änderungen zwischen den Einzelbildern, denen die gleiche Schwerpunktwellenlänge zugeordnet ist, in den Bildpaaren und
- Auswerten der Bilddaten und Verwenden der Änderungen zum Korrigieren von Bewegungen des Objektes (14) in den Bilddaten.

2. Verfahren nach Anspruch 1, wobei in mehreren Beleuchtungspulspaaren (10.L0, 10L1; 10.L0, 10.L2) genau eine Schwerpunktwellenlänge eines der Beleuchtungspulse (10.L0) des unmittelbar vorhergehenden der Beleuchtungspulspaare (10.L0, 10L1; 10.L0, 10.L2) wiederholt wird.

3. Verfahren nach Anspruch 2, wobei dieselbe Schwerpunktwellenlänge als Referenzschwerpunktwellenlänge wiederholt wird.

4. Verfahren nach einem der obigen Ansprüche, wobei ein zeitlicher Abstand (12) der Beleuchtungspulse der Beleuchtungspulspaare (10.L0, 10L1; 10.L0, 10.L2) so gewählt ist, dass sich die Einzelbilder jedes Bildpaares gegenüber dem Objekt (14) in der Ortslage um weniger als eine Specklekorn der Beleuchtung unterscheiden.

5. Verfahren nach einem der obigen Ansprüche, wobei eine Bewegung während der Abbildung des Objektes durch einen motorisch bewegten Objektträger realisiert wird.

6. Verfahren nach einem der obigen Ansprüche, wobei ein nichtlebendes in-vitro-Objekt abgebildet und dazu mit als Streuwellenbeleuchtung ausgebildeter Beleuchtungsstrahlung (B) beleuchtet wird, wobei mehrere Wellenlängen-Bildstapel aufgenommen werden und eine Verschiebung zwischen Beleuchtungsspecklen und dem Objekt durch eine motorische Bewegung des Objekts erreicht wird.

7. Verfahren nach einem der obigen Ansprüche, wobei diese motorische Bewegung des Objekts nur zwischen den Bildstapeln und nicht innerhalb der Bildstapel ausgeführt wird.

8. Verfahren nach einem der obigen Ansprüche, wobei auf dem Detektor (22) die Messstrahlung mit Referenzstrahlung überlagert wird, wobei für die Einzelbilder jedes Bildpaares unterschiedliche Referenzstrahlungsrichtungen vorgesehen werden, und die zwei Einzelbilder in einer Bildauswertung auf Basis der Referenzstrahlungsrichtungen separiert werden.

9. Weitfeld-Swept-Source-OCT zur Abbildung eines bewegten Objekts (14), insbesondere der Vorderkammer (16) des menschlichen Auges, wobei der OCT (12) aufweist:
- eine Strahlungsquelle (18), die in der Wellenlänge durchgestimmte Beleuchtungsstrahlung (B) abgibt, welche einzelne Beleuchtungspulse (10.L0, 10.L1) unterschiedlicher Schwerpunktwellenlänge aufweist, wobei die Beleuchtungspulse (10.L0, 10,L1, 10.L2) als Serie von Beleuchtungspulspaaren (10.L0, 10L1; 10.L0, 10.L2) abgegeben werden, die jeweils aus einem ersten Beleuchtungspuls (10.L0) und einem zweiten Beleuchtungspuls (10.L1, 10.L2) bestehen, wobei in den Beleuchtungspulspaaren (10.L0, 10L1; 10.L0, 10.L2) sich die Schwerpunktwellenlänge des ersten Beleuchtungspulses (10.L0) von der Schwerpunktwellenlänge des zweiten Beleuchtungspulses (10L1, 10.L2) unterscheidet und in mehreren der Beleuchtungspulspaare (10.L0, 10L1; 10.L0, 10.L2) mind. eine Schwerpunktwellenlänge eines der Beleuchtungspulse (10.L0) eines vorhergehenden der Beleuchtungspulspaare (10.L0, 10L1; 10.L0, 10.L2) wiederholt ist,
- einen 2D-Detektor (22), der einen Bildaufnahmezyklus umfassend eine Abfolge (2) von Belichtungsintervallen (4.1, 4.2, 4.3, 4.4) und Ausleseintervallen (6.1, 6.2, 6.3) ausführt,
- einen Strahlengang (24) zum Beleuchten des Objekts (14) mit der Beleuchtungsstrahlung (B) und zum Abbilden des beleuchteten Objekt (14) auf den 2D-Detektor (22) und
- eine Steuereinrichtung (C), die den 2D-Detektor (22) und die Strahlungsquelle (18) ansteuert und konfiguriert ist, Strahlungsquelle (18) und den 2D-Detektor (22) derart zu synchronisieren, dass die Beleuchtungspulspaare (10.L0, 10L1; 10.L0, 10.L2) um jedes zweite (6.1, 6.3) der Ausleseintervalle (6.1, 6.2, 6.3) der Abfolge (2) herum gruppiert sind und für jedes Beleuchtungspulspaar (10.L0, 10L1; 10.L0, 10.L2) der erste Beleuchtungspuls (10.L0) während eines letzten Drittels eines der Belichtungsintervalle (4.1, 4.2, 4.3, 4.4) und der zweite Beleuchtungspuls (10L1; 10.L2) während eines ersten Drittels des folgenden der Belichtungsintervalle (4.1, 4.2, 4.3, 4.4) abgegeben wird,
- wobei die Steuereinrichtung (C) weiter konfiguriert ist
-- zum Auslesen von Bilddaten für jedes Belichtungsintervall (4.1, 4.2, 4.3, 4.4) des Detektors (22) und zum Zuordnen der Bilddaten zu den Schwerpunktwellenlängen des im jeweiligen Belichtungsintervall abgegebenen Beleuchtungspulses (10.L0, 10,L1, 10.L.2) und zum Erzeugen von Bildpaaren entsprechend den Beleuchtungspulspaaren (10.L0, 10L1; 10.L0, 10.L2),
-- zum Ermitteln von Änderungen zwischen den Bilddaten der über die Beleuchtungspulspaare (10.L0, 10L1; 10.L0, 10.L2) hinweg wiederholten Beleuchtungspulse (10.L0) mit gleicher Schwerpunktwellenlänge und
-- zum Auswerten der Bilddaten und zum Verwenden der Änderungen zum Korrigieren von Bewegungen des Objektes (14) in den Bilddaten.

10. OCT nach Anspruch 9, wobei die Strahlungsquelle (18) einen Swept-Source Laser (42) aufweist, der eine Durchstimmwiederholrate aufweist, welche nicht niedriger ist, als eine durch Dauer von Belichtungsintervall (4.1, 4.2, 4.3, 4.4) und Ausleseintervall (6.1, 6.2, 6.3) definierte Bildwiederholfrequenz des Detektors (22).

11. OCT nach Anspruch 9 oder 10, wobei die Strahlungsquelle (18) einen von der Steuereinrichtung (C) zum Synchronisieren angesteuerten optischen Schalter (46) oder Umschalter (52) aufweist.

12. OCT nach einem der Ansprüche 9 bis 11, wobei eine Kohärenz-Tiefenauflösung besser oder gleich einer durch den Strahlengang definierten Schärfentiefe ist.

## Claims

1. A wide-field swept-source OCT method for imaging a moving object (14), in particular the anterior chamber (16) of the human eye, wherein the method comprises the following steps:
- providing illumination radiation (B), which is tuned in the wavelength and comprises individual illumination pulses (10.L0, 10.L1) of different centroid wavelength, wherein the illumination pulses (10.L0, 10,L1, 10.L2) are provided as a series of illumination pulse pairs (10.L0, 10L1; 10.L0, 10.L2), each consisting of a first illumination pulse (10.L0) and a second illumination pulse (10.L1, 10.L2), wherein in the illumination pulse pairs (10.L0, 10L1; 10.L0, 10.L2) the centroid wavelength of the first illumination pulse (10.L0) differs from the centroid wavelength of the second illumination pulse (10L1, 10.L2), and in a plurality of the illumination pulse pairs (10.L0, 10L1; 10.L0, 10.L2) at least one centroid wavelength of one of the illumination pulses (10.L0) of a preceding one of the illumination pulse pairs (10.L0, 10.L1; 10.L0, 10.L2) is repeated,
- illuminating the object (14) with the illumination radiation (B) and imaging the illuminated object (14) onto a 2D detector (22),
- operating the detector (22) according to an image recording cycle comprising a sequence (2) of exposure intervals (4.1, 4.2, 4.3, 4.4) and readout intervals (6.1, 6.2, 6.3),
- synchronizing the emission of the illumination pulses (10.L0, 10,L1, 10.L.2) and the operation of the detector (22) in such a way that the illumination pulse pairs (10.L0, 10L1; 10.L0, 10.L2) are grouped around every second one (6.1, 6.3) of the readout intervals (6.1, 6.2, 6.3) of the sequence (2) and, for each illumination pulse pair (10.L0, 10L1; 1 0.L0, 1 0.L2), the first illumination pulse (10.L0) is emitted during a last third of one of the exposure intervals (4.1, 4.2, 4.3, 4.4) and the second illumination pulse (10L1; 10.L2) is emitted during a first third of the next of the exposure intervals (4.1, 4.2, 4.3, 4.4), and
- reading image data of each exposure interval (4.1, 4.2, 4.3, 4.4) of the detector (22) and assigning the image data to the centroid wavelengths of the illumination pulse (10.L0, 10,L1, 10.L.2) emitted in the respective exposure interval, wherein image pairs consisting of single images are generated according to the illumination pulse pairs (10.L0, 10L1; 10.L0, 10.L2),
- determining changes in the image pairs between the single images to which the same centroid wavelength is assigned, and
- evaluating the image data and using the changes to correct movements of the object (14) in the image data.

2. The method as claimed in claim 1, wherein in a plurality of illumination pulse pairs (10.L0, 10L1 ; 10.L0, 10.L2) exactly one centroid wavelength of one of the illumination pulses (10.L0) of the immediately preceding one of the illumination pulse pairs (10.L0, 10L1; 10.L0, 10.L2) is repeated.

3. The method as claimed in claim 2, wherein the same centroid wavelength is repeated as the reference centroid wavelength.

4. The method as claimed in any of the above claims, wherein a temporal distance (12) of the illumination pulses of the illumination pulse pairs (10.L0, 10L1; 10.L0, 10.L2) is selected such that the single images of each image pair differ in location from the object (14) by less than one speckle grain of the illumination.

5. The method as claimed in any of the above claims, wherein a movement during the imaging of the object is realized by a motorically moved object carrier.

6. The method as claimed in any of the above claims, wherein a non-living in-vitro object is imaged and illuminated for this purpose with illumination radiation (B) formed as scattering wave illumination, wherein a plurality of wavelength image stacks are recorded and a displacement between illumination speckles and the object is achieved by a motorized movement of the object.

7. The method as claimed in any of the above claims, wherein this motorized movement of the object is carried out only between the image stacks and not within the image stacks.

8. The method as claimed in any of the above claims, wherein the measurement radiation is superimposed with reference radiation on the detector (22), wherein different reference radiation directions are provided for the single images of each image pair, and the two single images are separated in an image evaluation based on the reference radiation directions.

9. A wide-field swept-source OCT for imaging a moving object (14), in particular the anterior chamber (16) of the human eye, wherein the OCT (12) comprises:
- a radiation source (18), which emits illumination radiation (B) which is tuned in the wavelength and comprises individual illumination pulses (10.L0, 10.L1) of different centroid wavelength, wherein the illumination pulses (10.L0, 10,L1, 10.L2) are emitted as a series of illumination pulse pairs (10.L0, 10L1; 10.L0, 10.L2), each consisting of a first illumination pulse (10.L0) and a second illumination pulse (10.L1, 10.L2), wherein in the illumination pulse pairs (10.L0, 10L1 ; 10.L0, 10.L2) the centroid wavelength of the first illumination pulse (10.L0) differs from the centroid wavelength of the second illumination pulse (10L1, 10.L2), and in a plurality of the illumination pulse pairs (10.L0, 10L1; 10.L0, 10.L2) at least one centroid wavelength of one of the illumination pulses (10.L0) of a preceding one of the illumination pulse pairs (10.L0, 10L1; 10.L0, 10.L2) is repeated,
- a 2D detector (22), which performs an image recording cycle comprising a sequence (2) of exposure intervals (4.1, 4.2, 4.3, 4.4) and readout intervals (6.1, 6.2, 6.3),
- a beam path (24) for illuminating the object (14) with the illumination radiation (B) and for imaging the illuminated object (14) onto the 2D detector (22), and
- a control device (C), which controls the 2D detector (22) and the radiation source (18) and is configured to synchronize the radiation source (18) and the 2D detector (22) in such a way that the illumination pulse pairs (10.L0, 10L1; 10.L0, 10.L2) are grouped around every second one (6.1, 6.3) of the readout intervals (6.1, 6.2, 6.3) of the sequence (2) and, for each illumination pulse pair (10.L0, 10L1; 10.L0, 10.L2), the first illumination pulse (10.L0) is emitted during a last third of one of the exposure intervals (4.1, 4.2, 4.3, 4.4) and the second illumination pulse (10L1; 10.L2) is emitted during a first third of the next of the exposure intervals (4.1, 4.2, 4.3, 4.4),
- wherein the control device (C) is further configured
-- for reading image data for each exposure interval (4.1, 4.2, 4.3, 4.4) of the detector (22) and for assigning the image data to the centroid wavelengths of the illumination pulse (10.L0, 10,L1, 10.L.2) emitted in the respective exposure interval, and for generating image pairs according to the illumination pulse pairs (10.L0, 10L1; 10.L0, 10.L2),
-- for determining changes between the image data of the illumination pulses (10.L0) with the same centroid wavelength repeated across the illumination pulse pairs (10.L0, 10L1; 10.L0, 10.L2), and
-- for evaluating the image data and for using the changes to correct movements of the object (14) in the image data.

10. The OCT as claimed in claim 9, wherein the radiation source (18) comprises a swept-source laser (42), which has a tuning repetition rate which is not lower than a frame rate of the detector (22) defined by the duration of the exposure interval (4.1, 4.2, 4.3, 4.4) and readout interval (6.1, 6.2, 6.3).

11. The OCT as claimed in claim 9 or 10, wherein the radiation source (18) comprises an optical switch (46) or switch (52) controlled by the control device (C) for synchronization.

12. The OCT as claimed in any of claims 9 to 11, wherein a coherence depth resolution is better than or equal to a depth of field defined by the beam path.

## Revendications

1. Procédé de TCO à source balayée à champ large d'imagerie d'un objet en déplacement (14), en particulier de la chambre antérieure (16) de l'œil humain, le procédé présentant les étapes suivantes :
- mise à disposition d'un rayonnement d'éclairage (B) réglé en longueur d'onde, qui présente des impulsions d'éclairage individuelles (10.L0, 10.L1) de différentes longueurs d'onde centrales, les impulsions d'éclairage (10.L0, 10.L1, 10.L2) étant mises à disposition sous la forme d'une série de paires d'impulsions d'éclairage (10.L0, 10L1 ; 10.L0, 10.L2), qui consistent chacune en une première impulsion d'éclairage (10.L0) et une deuxième impulsion d'éclairage (10.L1, 10.L2), dans lequel, dans les paires d'impulsions d'éclairage (10.L0, 10L1 ; 10.L0, 10.L2), la longueur d'onde centrale de la première impulsion d'éclairage (10.L0) est différente de la longueur d'onde centrale de la deuxième impulsion d'éclairage (10L1, 10.L2) dans plusieurs des paires d'impulsions d'éclairage (10.L0, 10L1 ; 10.L0, 10.L2), au moins une longueur d'onde centrale d'une des impulsions d'éclairage (10.L0) d'une précédente paire des paires d'impulsions d'éclairage (10.L0, 10.L1 ; 10.L0, 10.L2) est répétée,
- éclairage de l'objet (14) avec le rayonnement d'éclairage (B) et imagerie de l'objet éclairé (14) sur un détecteur 2D (22),
- fonctionnement du détecteur (22) selon un cycle d'acquisition d'images comprenant une séquence (2) d'intervalles d'exposition (4.1, 4.2, 4.3, 4.4) et d'intervalles de lecture (6.1, 6.2, 6.3),
- synchronisation de l'émission des impulsions d'éclairage (10L0, 10L1, 10L2) et du fonctionnement du détecteur (22), de telle sorte que les paires d'impulsions d'éclairage (10L0, 10L1 ; 10.L0, 10.L2) sont regroupées autour de chaque deuxième (6.1, 6.3) des intervalles de lecture (6.1, 6.2, 6.3) de la séquence (2) et pour chaque paire d'impulsions d'éclairage (10.L0, 10L1 ; 10.L0, 10.L2), la première impulsion d'éclairage (10.L0) est émise pendant un dernier tiers de l'un des intervalles d'exposition (4.1, 4.2, 4.3, 4.4) et la deuxième impulsion d'éclairage (10L1 ; 10.L2) est émise pendant un premier tiers de l'intervalle d'exposition suivant (4.1, 4.2, 4.3, 4.4), et
- lecture des données d'image de chaque intervalle d'exposition (4.1, 4.2, 4.3 et 4.4) du détecteur (22) et attribution des données d'image aux longueurs d'onde centrales de l'impulsion d'éclairage (10.L0, 10.L1, 10.L2) émise dans l'intervalle d'exposition respectif, dans lequel sont générées des paires d'images composées d'images individuelles correspondant aux paires d'impulsions d'éclairage (10.L0, 10L1 ; 10.L0, 10.L2) ;
- détermination de variations entre les images individuelles auxquelles la même longueur d'onde centrale est attribuée dans les paires d'images ; et
- évaluation des données d'image et utilisation des variations pour corriger des mouvements de l'objet (14) dans les données d'image.

2. Procédé selon la revendication 1, dans lequel, dans plusieurs paires d'impulsions d'éclairage (10.L0, 10L1 ; 10.L0, 10.L2), précisément une longueur d'onde centrale de l'une des impulsions d'éclairage (10.L0) de la paire d'impulsions d'éclairage (10.L0, 10L1 ; 10.L0, 10.L2) immédiatement précédente est répétée.

3. Procédé selon la revendication 2, dans lequel la même longueur d'onde centrale est répétée en tant que longueur d'onde centrale de référence.

4. Procédé selon l'une des revendications précédentes, dans lequel un intervalle de temps (12) entre les impulsions d'éclairage des paires d'impulsions d'éclairage (10.L0, 10L1 ; 10.L0, 10.L2) est choisi de telle sorte que les images individuelles de chaque paire d'images diffèrent de moins d'un grain de chatoiement de l'éclairage par rapport à l'objet (14) dans la position locale.

5. Procédé selon l'une des revendications précédentes, dans lequel un mouvement pendant l'imagerie de l'objet est réalisé par un support d'objet à entraînement motorisé.

6. Procédé selon l'une des revendications précédentes, dans lequel un objet in vitro non vivant est représenté et éclairé à cet effet par un rayonnement d'éclairage (B) conçu comme un éclairage à ondes diffusées, dans lequel plusieurs piles d'images de différentes longueurs d'onde sont enregistrées et un déplacement entre les taches d'éclairage et l'objet est obtenu par un mouvement motorisé de l'objet.

7. Procédé selon l'une des revendications précédentes, dans lequel ce mouvement motorisé de l'objet n'est effectué qu'entre les piles d'images et non à l'intérieur des piles d'images.

8. Procédé selon l'une des revendications précédentes, dans lequel le rayonnement de mesure est superposé au rayonnement de référence sur le détecteur (22), dans lequel différentes directions de rayonnement de référence sont prévues pour les images individuelles de chaque paire d'images, et les deux images individuelles sont séparées dans une évaluation d'images sur la base des directions de rayonnement de référence.

9. TCO à source balayée à champ large pour l'imagerie d'un objet en déplacement (14), en particulier de la chambre antérieure (16) de l'œil humain, la TCO (12) comportant :
- une source de rayonnement (18) qui émet un rayonnement d'éclairage (B) réglé en longueur d'onde, qui présente des impulsions d'éclairage individuelles (10.L0, 10.L1) de différentes longueurs d'onde centrales, les impulsions d'éclairage (10.L0, 10.L1, 10.L2) étant émises sous la forme d'une série de paires d'impulsions d'éclairage (10.L0, 10L1 ; 10.L0, 10.L2), qui consistent chacune en une première impulsion d'éclairage (10.L0) et une deuxième impulsion d'éclairage (10.L1, 10.L2), dans les paires d'impulsions d'éclairage (10.L0, 10L1 ; 10.L0, 10.L2), la longueur d'onde centrale de la première impulsion d'éclairage (10.L0) étant différente de la longueur d'onde centrale de la deuxième impulsion d'éclairage (10L1, 10.L2) et dans plusieurs des paires d'impulsions d'éclairage (10.L0, 10L1 ; 10.L0, 10.L2), au moins une longueur d'onde centrale d'une des impulsions d'éclairage (10.L0) d'une précédente paire des paires d'impulsions d'éclairage (10.L0, 10.L1 ; 10.L0, 10.L2) étant répétée,
- un détecteur 2D (22), qui exécute un cycle d'acquisition d'images comprenant une séquence (2) d'intervalles d'exposition (4.1, 4.2, 4.3, 4.4) et d'intervalles de lecture (6.1, 6.2, 6.3),
- un chemin de rayonnement (24) pour éclairer l'objet (14) avec le rayonnement d'éclairage (B) et pour reproduire l'objet éclairé (14) sur le détecteur 2D (22) et,
- un dispositif de commande (C) qui commande le détecteur 2D (22) et la source de rayonnement (18) et qui est configuré pour synchroniser la source de rayonnement (18) et le détecteur 2D (22) de telle sorte que les paires d'impulsions d'éclairage (10L0, 10L1 ; 10.L0, 10.L2) sont regroupées autour de chaque deuxième (6.1, 6.3) des intervalles de lecture (6.1, 6.2, 6.3) de la séquence (2) et pour chaque paire d'impulsions d'éclairage (10.L0, 10L1 ; 10.L0, 10.L2), la première impulsion d'éclairage (10.L0) est émise pendant un dernier tiers d'un des intervalles d'exposition (4.1, 4.2, 4.3, 4.4) et la deuxième impulsion d'éclairage (10L1 ; 10.L2) est émise pendant un premier tiers de l'intervalle d'exposition suivant (4.1, 4.2, 4.3, 4.4), et
- le dispositif de commande (C) étant en outre configuré
-- pour lire des données d'image pour chaque intervalle d'exposition (4.1, 4.2, 4.3 et 4.4) du détecteur (22) et pour attribuer les données d'image aux longueurs d'onde centrales de l'impulsion d'éclairage (10.L0, 10.L1, 10.L2) émise dans l'intervalle d'exposition respectif, et pour générer des paires d'images correspondant aux paires d'impulsions d'éclairage (10.L0, 10L1 ; 10.L0, 10.L2),
-- pour déterminer des variations entre les données d'image des impulsions d'éclairage (10.L0) répétées à travers les paires d'impulsions d'éclairage (10.L0, 10L1 ; 10.L0, 10.L2) ayant la même longueur d'onde centrale et
-- pour évaluer les données d'image et pour utiliser les variations pour corriger les mouvements de l'objet (14) dans les données d'image.

10. TCO selon la revendication 9, dans laquelle la source de rayonnement (18) présente un laser à source balayée (42), qui présente une fréquence de répétition de balayage qui n'est pas inférieure à une fréquence de répétition d'image du détecteur (22) définie par la durée de l'intervalle d'exposition (4.1, 4.2, 4.3, 4.4) et de l'intervalle de lecture (6.1, 6.2, 6.3).

11. TCO selon la revendication 9 ou 10, dans laquelle la source de rayonnement (18) présente un interrupteur (46) ou commutateur (52) optique commandé par le dispositif de commande (C) pour la synchronisation.

12. TCO selon l'une des revendications 9 à 11, dans laquelle une résolution de profondeur de cohérence est supérieure ou égale à une profondeur de champ définie par le chemin de rayonnement.
